(19) Europäisches Patentamt
European Patent Office
Office européen des brevets

(11) Publication number: **0 302 392**
**A2**

(12) # EUROPEAN PATENT APPLICATION

(21) Application number: 88112234.5

(22) Date of filing: 28.07.88

(51) Int. Cl.4: C07C 43/12 , C07C 43/17

(30) Priority: 03.08.87 JP 193941/87

(43) Date of publication of application:
08.02.89 Bulletin 89/06

(84) Designated Contracting States:
DE FR GB IT

(71) Applicant: **DAIKIN INDUSTRIES LIMITED**
Umeda Center Building 4-12, Nakazaki-nishi
2-chome Kita-ku
Osaka-shi Osaka-fu(JP)

(72) Inventor: **Furutaka, Yasuhisa**
18-5, Midorigaoka 1-chome
Takatsuki-shi Osaka-fu(JP)
Inventor: **Honda, Tsunetoshi**
2-21-21, Hitotsuya
Settsu-shi Osaka-fu(JP)
Inventor: **Iwashita, Nobuyoshi**
2-21-21, Hitotsuya
Settsu-shi Osaka-fu(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **Polyfluoroethers.**

(57) A polyether of the formula:

EP 0 302 392 A2

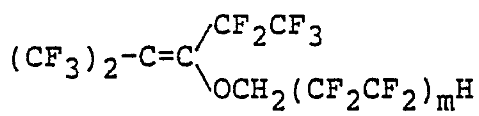

(I)

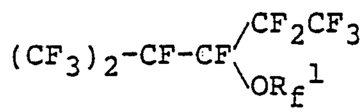

(II)

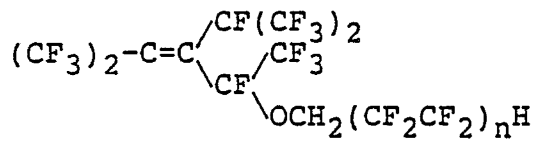

(III)

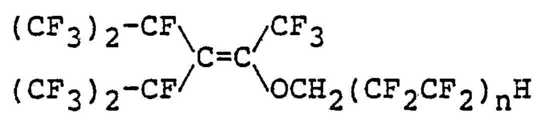

(IV)

or

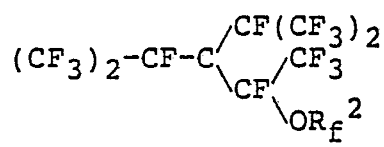

(V)

wherein m is a number of 2 to 5, n is a number of 1 to 5, $R_f^1$ is a perfluoroalkyl group having 7 to 11 carbon atoms, and $R_f^2$ is a perfluoroalkyl group having 3 to 11 carbon atoms, which has a high boiling point and is chemically and thermally stable.

# POLYFLUOROETHERS

## BACKGROUND OF THE INVENTION

### Field of the Invention

The present invention relates to polyfluoroethers. More particularly, the present invention relates to polyfluoroethers which are useful as testing liquids for integrated circuits, heating media for gas phase soldering and the like.

### Description of the Related Art

Polyfluoroethers are known from U.S. Patent No. 2,500,388, J. Org. Chem., 50 (1985) 5156 and Japanese Patent Kokai Publication No. 43934/1983. Since the known polyfluoroethers have low boiling points and their production costs are high, they are not suitable as the testing liquids for integrated circuits and the heating media for gas phase soldering.

## SUMMARY OF THE INVENTION

One object of the present invention is to provide a chemically and thermally stable polyfluoroether having a boiling point in a range from about 200 °C to about 250 °C.

Another object of the present invention is to provide a process for preparing the polyfluoroether in a high yield.

Accordingly, the present invention provides a polyether of the formula:

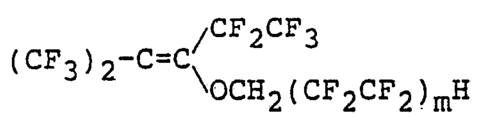

(I)

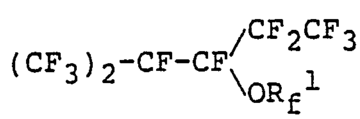

(II)

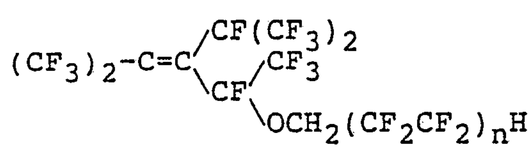

(III)

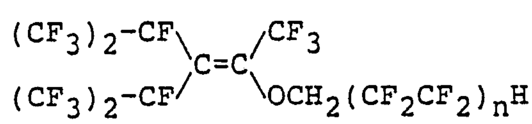

(IV)

or

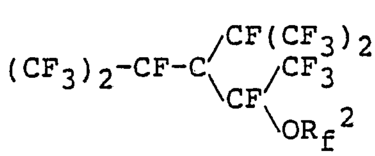

(V)

wherein m is a number of 2 to 5, preferably 3 and 4, n is a number of 1 to 5, preferably 2 to 4, $R_f^1$ is a perfluoroalkyl group having 7 to 11 carbon atoms, preferably 7 to 9 carbon atoms, and $R_f^2$ is a

perfluoroalkyl group having 3 to 11 carbon atoms, preferably 5 to 9 carbon atoms.

## DETAILED DESCRIPTION OF THE INVENTION

The polyfluoroether according to the present invention may be prepared as follows:

The polyfluoroether (I) can be prepared by reacting a dimer of hexafluoropropene, namely perfluoro(2-methyl-2-pentene) with a fluorine-containing alcohol of the formula:

$H(CF_2CF_2)_mCH_2OH$ (VI)

wherein m is the same as defined above in the presence of a base (cf. Bulletin of Organic Synthesis Society, 39 (1) 51 (1981)).

The polyfluoroether (II) can be prepared by the fluorination of the polyfluoroether (I) with fluorine gas.

The polyfluoroether (III) or (IV) can be prepared by reacting a trimer of hexafluoropropene, namely perfluoro[3-(1-methylethyl)-4-methyl-2-pentene] with a fluorine-containing alcohol of the formula:

$H(CF_2CF_2)_nCH_2OH$ (VII)

wherein n is the same as defined above in the presence of a base.

By fluorination, the polyfluoroether (III) or (IV) can be converted to a perfluoroether such as the polyfluoroether (V).

In the above reaction, the fluorine-containing alcohol (VI) or (VII) is used in an amount of 0.5 to 1.2 moles, preferably 0.8 to 1.0 mole per mole of the dimer or trimer of hexafluoropropene.

The base is used in an amount of 0.8 to 10 moles, preferably 1 to 3 moles per mole of the fluorine-containing alcohol. Specific examples of the base are tertiary amines such as triethylamine.

The reaction temperature is from -10 to +10 °C.

In the fluorination, the fluorine gas is used in an amount of 1 to 20 moles, preferably 1.5 to 5 moles per mole of the polyfluoroether (I), (III) or (IV).

The fluorine gas may diluted with an inert gas such as nitrogen and helium to a concentration of 5 to 90 % by volume, preferably 20 to 60 % by volume, although the fluorine gas can be used without dilution. When the fluorine gas is irradiated by ultraviolet light generated by, for example, a high pressure or low pressure mercury lamp, more radicals are formed, whereby the yield of the perfluoroether is improved.

The fluorination temperature is generally from -70 °C to +100 °C, preferably from -30 °C to +25 °C.

The fluorination can be carried out in the presence of a solvent such as perfluoroalkanes (e.g. perfluorohexane, perfluoroctane, etc.).

The present invention will be illustrated by following Examples.

### Example 1

To a 500 ml flask equipped with a dropping funnel, a thermometer and a reflux condenser, perfluoro(2-methyl-2-pentene) (200 g, 0.67 mol) and triethylamine (84 g, 0.83 mol) were added. Thereto, a fluorine-containing alcohol of the formula: $H(CF_2CF_2)_3CH_2OH$ (184 g, 0.56 mol) was dropwise added over 6 hours while cooling by ice at a temperature of 0 to 5 °C.

The reaction mixture was stirred at 25 °C for 10 hours and poured into iced water. The oil phase was washed with dilute hydrochloric acid and then water, dried over calcium chloride and distilled under reduced pressure to obtain a colorless liquid. Boiling point, 103-105 °C/25 mmHg.

By NMR and mass spectrometry, the liquid was identified as a mixture of a compound of the formula:

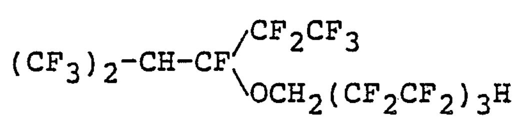

and a compound of the formula:

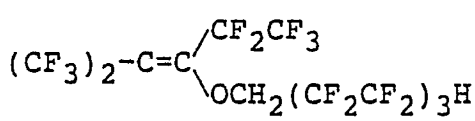

in a molar ratio of about 1:1.

The product mixture was refluxed together with a 50 % aqueous solution of KOH (100 ml) and diisobutylamine (50 ml) for 20 hours to convert the former saturated compound to the latter unsaturated compound. Boiling point, 210-215 °C.

Example 2

In an iron-made reactor equipped with a cooling jacket and having a sapphire made window through which UV light was irradiated, perfluoroisohexane (550 g) as a solvent was added. While irradiating the reactor by a 75 W high pressure mercury lamp (manufactured by Toshiba), fluorine gas diluted with nitrogen to a concentration of 20 % by volume was introduced in the reactor at a rate of 60 ml/min. at a temperature of 0 to 5 °C for 10 minutes. Thereafter, while irradiating the reactor by the mercury lamp and intro ducing the diluted fluorine gas under the same conditions as above, the mixture obtained in Example 1 was supplied to the reactor at a rate of 1.24 g/hr. After 10 hours, the supply of the mixtures was stopped and the diluted fluorine gas was introduced for another 1 hour.

After purging the interior of the reactor for 30 minutes, the reaction mixture was washed with a 10 % aqueous solution of sodium hydroxide and then water, dried over calcium chloride and distilled to obtain 3-n-heptoxy-2-methylpentane (8.6 g) of the formula:

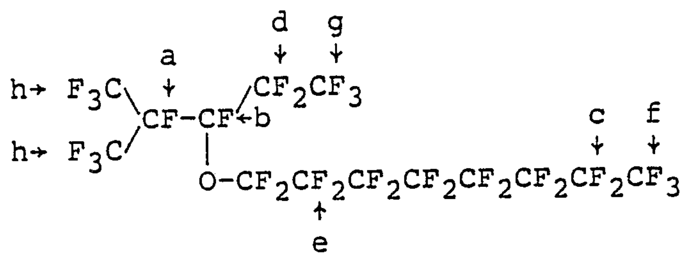

Boiling point, 198-200 °C.

MS: M/e = 385 ($M^+$-$C_6F_{13}$), 369 ($M^+$-$C_6F_{13}O$), 319 ($M^+$-$OC_7F_{15}$).

$^{19}$F-NMR (internal standard: trifluoroacetic acid) (the positions of the fluorine atoms being indicated by the alphabets "a" to "h" in the above formula)

δ (ppm) = -104.2 (a), -55.6 (b), -49.6 (c), -47.7 (d), -41.4 (e), -4.9 (f), -3.7 (g), 4.7 (h).

Example 3

In the same manners as in Examples 1 and 2 but using a fluorine-containing alcohol of the formula: $H-(CF_2CF_2)_4CH_2OH$ as the fluorine-containing alcohol, the reactions were carried out to obtain a compound of the formula:

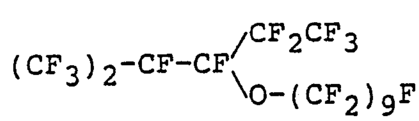

Boiling point, 221-222 °C.

Example 4

In the same manner as in Example 1 but using perfluoro[3-(1-methylethyl)-4-methyl-2-pentene] (302 g, 0.67 mol) in place of perfluoro(2-methyl-2-pentene), the reaction was carried. The reaction mixture was treated with an alkali/amine solution and rectified to obtain a compound of the formula:

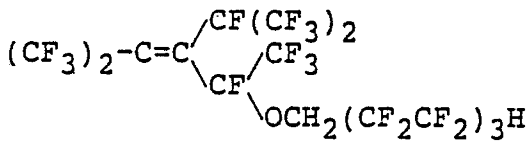

and a compound of the formula:

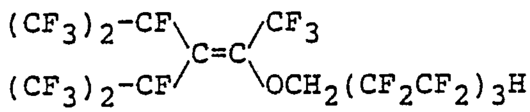

having a boiling point of 230 to 235 °C. Total yield, 700 g.

These compounds were thermally stable.

**Claims**

1. A polyether of the formula:

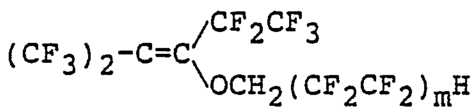
(I)

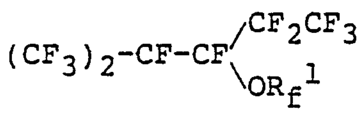
(II)

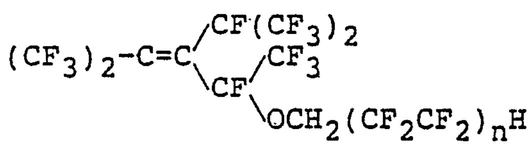
(III)

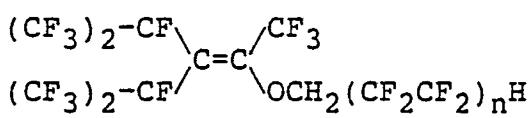
(IV)

or

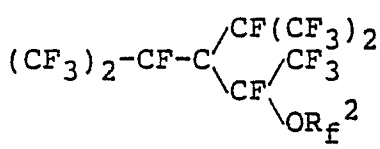
(V)

wherein m is a number of 2 to 5, n is a number of 1 to 5, $R_f^1$ is a perfluoroalkyl group having 7 to 11 carbon atoms, and $R_f^2$ is a perfluoroalkyl group having 3 to 11 carbon atoms.

2. A polyether of the formula:

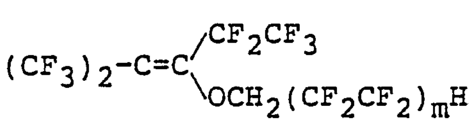
(I)

wherein m is as defined above.

3. A polyether of the formula:

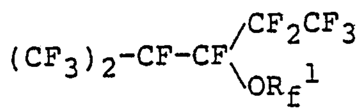

(II)

wherein $R_f^1$ is as defined above.

4. A polyether of the formula:

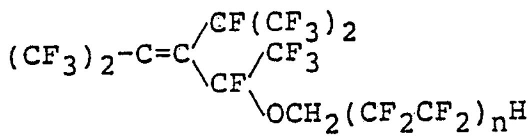

(III)

wherein n is as defined above.

5. A polyether of the formula:

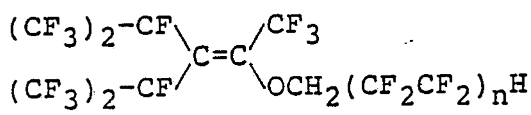

(IV)

wherein n is as defined above.

6. A polyether of the formula:

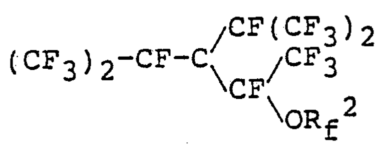

(V)

wherein $R_f^2$ is as defined above.